# EUROPEAN PATENT SPECIFICATION

(11) **EP 3 326 659 B1**
(45) Date of publication and mention of the grant of the patent: **13.11.2019**
(21) Application number: 16200370.1
(22) Date of filing: 23.11.2016
(51) Int. Cl.: A61L 15/46, A61F 13/84

(54) **AN ABSORBENT ARTICLE COMPRISING AN ODOR CONTROL MIXTURE**
SAUGFÄHIGER ARTIKEL MIT EINEM DUFTSTEUERUNGSGEMISCH
ARTICLES ABSORBANTS COMPRENANT UN MÉLANGE DE CONTRÔLE DES ODEURS

(43) Date of publication of application: 30.05.2018
(73) Proprietor: Toiletry Sales Limited, Crigglestone WF4 3HT (GB)
(72) Inventor: WALKER, Richard John, Crigglestone WF4 3HT (GB); SIERRI, Giancarlo, 64030 Montefino (IT); RANDALL, Keith, Crigglestone WF4 3HT (GB)
(74) Representative: Fiussello, Francesco

(56) References cited:
- EP-A1- 1 842 564
- WO-A1-96/04938
- WO-A2-98/26808
- US-A1- 2010 111 889
- DATABASE WPI Week 201653 Thomson Scientific, London, GB; AN 2016-352691 XP002770045, & CN 105 561 737 A (SHENZHEN XINGNENGBAO ENVIRONMENTAL TECHN) 11 May 2016 (2016-05-11)

## Description

### FIELD OF THE INVENTION

The present invention relates to absorbent articles which are provided with an odor control system. In particular the odor control system comprises an odor control mixture.

### BACKGROUND OF THE INVENTION

Absorbent articles and particularly disposable absorbent articles of personal hygiene are known in the art. Typical examples include sanitary napkins, panty liners, adult incontinence articles, diapers, paper towels, tampon, interlabial products, perspiration pads, training pants, incontinence underwear, incontinence pull-ons, bed underpad.

Such articles are often used to absorb and retain bodily fluids and other exudates excreted by the human body. Typically, such exudates are perceived as malodorous and offensive. Therefore, methods and materials for controlling and reducing malodors in absorbent articles have been developed.

EP510619 discloses a wide variety of materials, which have proven to be effective in certain circumstances in reducing malodors in absorbent articles of personal hygiene.

EP959846 discloses similar materials comprising polyacrylate superabsorbers and silica.

EP811387 discloses absorbent articles being provided with a zeolite and silica odor control system.

EP963186 discloses an odor control systems comprising zeolites, silica and polyacrylic superabsorbers.

EP912149 discloses chelating agents for use in odor control in absorbent articles, particularly polyfunctionally substituted aromatic chelants.

US2010111889 discloses a malodor control system suitable for use in disposable articles, such as disposable cleaning wipes, baby wipes, or skin care wipes, is disclosed. The system may comprise an aldehyde, an ester, an ionone, and optionally a macrocyclic musk.

EP1842564 discloses an absorbent article comprising an odour control system which comprises aldehydes as a first class of odour control material that reduces odour by acting on a malodorous substance, and a second class of odour control material that inhibits the nose receptors. The odour control system may further comprise a highly volatile component such as terpineol, benzylacetate etc.

CN105561737 discloses an absorbent for biological deodorization and a preparation method of the absorbent. The absorbent for the biological deodorization is prepared from the following components in percentage by weight: 81 to 90 percent of water, 3.2 to 10 percent of organic acid, 0.1 to 0.23 percent of phenols, 4 to 8 percent of ketones, 0.2 to 0.7 percent of aldehydes, 0.1 to 0.15 percent of alcohols and 0.1 to 0.3 percent of esters. The absorbent disclosed by the invention has the beneficial effects that the problem of unsatisfied deodorization effect caused by defects of an existing biological deodorization absorption liquefying agent is solved, a comprehensive complexing agent which can be used for absorbing and liquefying various odor-causing matters is developed, and an effect of absorbing and liquefying odor matters is up to 60 percent to 85 percent.

WO9826808 discloses compositions and articles such as catamenials, diapers, pantiliners, adult incontinence garments, and underarm shields which minimize odor caused by body fluids and which optionally provide a pleasant scent signal to indicate that the odor is being removed. The odor control is provided by a combination of (1) material that inhibits the formation of odor that has at least one attribute selected from the group consisting of antimicrobial activity, urease inhibition activity, pH adjustment activity, and mixtures thereof; and (2) odor-absorbing material for objectionable odor molecules selected from the group consisting of: cyclodextrin; zeolite; activated carbon; kieselguhr; acid salt forming materials; and mixtures thereof. The scent signal is provided by cyclodextrin/perfume inclusion complexes and/or matrix perfume microcapsules to assure the wearer that the product is working.

WO9604938 discloses an aqueous composition for reducing malodor impression. The composition comprises from about 0.1 % to about 20 % by weight of the composition, of solubilized, water-soluble alkali metal salt selected from the group consisting of carbonate salts, bicarbonate salts, and mixtures thereof, from about 0.01 % to about 1 % by weight of the composition, of perfume. Optionally, but preferably, the composition comprises from about 0 % to about 5 % by weight of the composition, of solubilized, water-soluble cyclodextrin, and from about 0 % to about 3 % by weight of the composition, of solubilizing aid. The composition is essentially free of any material that would soil or stain fabric and contains less than about 5 % by weight of the composition, of low molecular weight monohydric alcohol, and has a pH of from about 7.5 to about 10.5.

All of the above solutions can provide a degree of malodor reduction in absorbent articles.

However, due to the nature of action and the materials chosen only a limited variety of malodorous compounds can be counteracted.

Therefore it is desirable to provide absorbent articles having an odor control system, which is capable to reduce a wide variety of malodors in a holistic manner.

### SUMMARY OF THE INVENTION

The aim of the present invention is therefore to provide for an absorbent article comprising an odor control system which is capable to reduce a wide variety of malodors and is easy and safe to manufacture, to store and to apply, in particular in contact with the skin and without any health concern.

According to the present invention the abovementioned aim is achieved by an absorbent article according to claim 1.

### BRIEF DESCRIPTION OF THE DRAWINGS

The present invention will also be described with reference to the accompanying drawings, in which:
- Figure 1 shows a graph of an odor testing with a first odor control mixture;
- Figure 2 shows a graph of an odor testing with a second odor control mixture; and
- Figure 3 shows a graph of an odor testing with a third odor control mixture.

### DETAILED DESCRIPTION OF THE INVENTION

The term "absorbent article" is used herein in a very broad sense including any article able to receive and/or absorb and/or contain and/or retain fluids and/or exudates, especially bodily fluids/bodily exudates.

Exemplary absorbent articles in the context of the present invention are disposable absorbent articles. The term "disposable" is used herein to describe articles, which are not intended to be laundered or otherwise restored or reused as an article, i.e. they are intended to be discarded after a single use and preferably to be recycled, composted or otherwise disposed of in an environmentally compatible manner. Typical examples include sanitary napkins, panty liners, adult incontinence articles, diapers, paper towels, tampon, interlabial products, perspiration pads, training pants, incontinence underwear, incontinence pull-ons, bed underpad, but also surgical and wound dressings. Certain absorbent articles include a fluid pervious topsheet, a fluid impervious backsheet that is preferably water vapour and/or gas pervious and an absorbent core comprised there between.

The term "use" refers to the period of time that starts when the absorbent article is actually put in contact with the anatomy of a wearer.

It is further underlined that the odor benefits provided by the absorbent articles of the present invention are already provided as soon as the absorbent articles is used.

By "body fluid" it is meant herein any fluid produced by human body including, but not limited to, perspiration, urine, menstrual fluids, vaginal secretions, faeces and the like.

According to the present invention the odor control system comprises an odor control mixture. The odor control mixture reduces the malodor generated by a broad range of odorous materials typically occurring in or resulting from the degradation of body fluids and/or materials making up the absorbent article.

Odor Control materials can reduce the malodor unpleasantness according to different mechanisms, e.g. they can reduce the amount of malodorous molecules through absorption/adsorption mechanisms and/or can react with the malodorous molecules transforming them into low volatile/non-odorous ones and/or can suppress malodorous molecules volatility and/or can prevent the malodor generation by inhibiting degradative processes caused by microorganisms metabolic activity and can reduce malodor by acting on the user's *nose* receptors.

Preferably the odor control mixture of the present invention comprises a specific selection of different materials and particularly a specific selection of three different compounds. It has been found by the present inventors that the combination of certain odor control materials results in a very effective in terms of odor reduction.

Specifically, the odor control mixture is very effective in terms of malodor reduction when combining at least one alcohol or phenol, at least one aldehyde and at least one acetate.

Particularly advantageous is an absorbent article comprising an odor control mixture comprising:
- at least one alcohol or phenol selected in the group a) consisting of 2-phenylethanol, terpineol, 3,7-dimethyloctan-3-ol, tetrahydro-2-isobutyl-4-methylpyran-4-ol, 2,6-di-tert-butyl-p-cresol, p-menth-1-en-8-ol, trimethylbenzenepropanol;
- at least one aldehyde selected in the group b) consisting of piperonal, alfa-methyl-1,3-benzodioxole-5-propionaldehyde, 3-p-cumenyl-2-methylpropionaldehyde, 6-methoxy-2,6-dimethylheptan-1-al, anisaldheyde;
- at least one acetate selected in the group c) consisting of benzyl acetate, 3,7-dimethyloctan-3-yl acetate, geranyl acetate, hexyl acetate, 2, 6-dimethyl-2-octyl acetate, citronellyl acetate.

Preferably the odor control mixture comprises:
- at least two alcohols or phenols selected in the group a),
- at least two aldehydes selected in the group b),
- at least two acetates selected in the group c). More preferably the odor control mixture comprises:
- at least four alcohols or fenol selected in the group a),
- at least two aldehydes selected in the group b),
- at least three acetates selected in the group c).

Particularly preferred are the odor control mixtures comprising two or more alcohols or phenols selected in the group consisting of 2-phenylethanol, 3,7-dimethyloctan-3-ol, tetrahydro-2-isobutyl-4-methylpyran-4-ol, mixed isomers (cis and trans), 2,6-di-tert-butyl-p-cresol.

More particularly preferred are the odor control mixtures comprising all the four alcohols or phenol 2-phenylethanol, 3,7-dimethyloctan-3-ol, tetrahydro-2-isobutyl-4-methylpyran-4-ol, mixed isomers (cis and trans), 2,6-di-tert-butyl-p-cresol.

Particularly preferred are the odor control mixtures comprising one or more aldehydes selected in the group consisting of alfa-methyl-1,3-benzodioxole-5-propionaldehyde, 6-methoxy-2,6-dimethylheptan-1-al.

More particularly preferred are the odor control mixtures comprising all the aldehydes alfa-methyl-1,3-benzodioxole-5-propionaldehyde, 6-methoxy-2,6-dimethylheptan-1-al.

Particularly preferred are the odor control mixtures comprising geranyl acetate.

Optimal results in term of stability and odor suppression have been obtained with odor control mixtures comprising the four alcohols or phenols 2-phenylethanol, 3,7-dimethyloctan-3-ol, tetrahydro-2-isobutyl-4-methylpyran-4-ol, mixed isomers (cis and trans), 2,6-di-tert-butyl-p-cresol; the aldehydes alfa-methyl-1,3-benzodioxole-5-propionaldehyde, 6-methoxy-2,6-dimethylheptan-1-al, and the acetate geranyl acetate.

Preferably the percentage quantity in weight of the alcohols of group a) in the odor control mixture is greater than the percentage quantity of the aldehydes of group b) or than the percentage quantity of the acetates of group c).

More preferably the percentage quantity in weight of the alcohols of group a) is between 8 and 24 %, the percentage quantity of the aldehydes of group b) is between 0.1 and 23 % and the percentage quantity of the acetates of group c) is between 1 and 8 %.

More preferably the relative percentages of groups a), b) and c) are between 40 and 90 for groups a), 3 and 45 for groups b) and 5 and 20 for groups c).

More preferably the relative percentages of groups a), b) and c) are between 60 and 70 for groups a), 15 and 25 for groups b) and 10 and 20 for groups c).

As a preferred example the relative percentages of groups a), b) and c) are 64,5%, 21,5% and 14%.

When the odor control mixture of the present invention is used in absorbent articles the individual odor control compounds can be employed at variable amounts.

Together with the abovementioned compounds contributing effectively to the odor suppression the odor control mixture usually comprises also solvents and additives. The skilled person modulates the quantity of the different non active ingredients of the odor control mixture according to the specific product needs and particularly according to the kind of absorbent material.

Further additional ingredients may include solvents as carriers for incorporating the odour control materials into the absorbent article. Suitable solvents are e.g benzyl-benzoate, isopropyl myristate, methyl abietate, ethanol, isopropanol, diethylene glycol monoethyl ether, glycerol, propylene glycol, 1,2-butylene glycol, dipropylene glycol, 2-methyl-2,4-pentanediol, diethyl phthalate, trietyl citrate, diethyl sebacate.

In case of an absorbent article the odor control mixture can be sprayed or coated or applied in any of the known methods to the part or the entire length of the product components preferably to internal layers as an acquisition layer between the topsheet and core.

The preferred quantity of odor control mixture in the absorbent article are between 1 and 180 mg, more preferably between 3 and 100 mg and even more preferably between 5 and 80 mg.

If quantities above 180 mg are used, the products are perceived as odorless after the test, but the production costs increase.

As a mere preferred example for an adult incontinent pad of Normal size and 275 mm long, 30 mg are preferably applied continuously on an acquisition distribution layer (AQL) 180 mm long and 3 mm wide. The above quantity is therefore distributed on an area of 180 mm x 3 mm = 0.00054 area sqm corresponding to 55 g/m2.

As a further preferred example in an adult Incontinent pad of sizes Extra and Extra Plus 335 mm long 38 mg can be applied continuously on an AQL and 230 mm long and 3 mm wide. The above quantity is therefore distributed on an area of 230 x 3 mm = 0.00069 area sqm, i.e. 55 g/m2.

For pantiliner for diapers for incontinence a total quantity of 1 mg to 180 mg can be advantageously applied.

The odor control mixture is preferably applied at the center of the acquisition distribution layer.

Alternatively the odor control mixture of the present invention can be present in any part of the absorbent article.

In one embodiment of the present invention the odor control mixture may be present in the absorbent core. In another embodiment of the present invention the odor control mixture may be present in any layer as an example in topsheet or any intermediate layer between the topsheet and the absorbent core and between the absorbent core and the backsheet.

Exemplary preferred compositions with particularly preferred quantities of the active principles are reported in Table 1.

**Table 1**

| Chemical Name | CAS-No. | Comp. 1 % wt. | Comp. 2 % wt. | Comp.3 % wt. |
|---|---|---|---|---|
| 2-phenylethanol | 60-12-8 | 3 - 10 | 3-10 | 3-10 |
| Terpineol | 8000-41-7 | 3 - 5 | 3-5 | |
| 3,7-dimethyloctan-3-ol | 78-69-3 | 1 - 3 | 3-5 | 3-5 |
| piperonal (heliotropine) | 120-57-0 | 0.1-10 | --- | 0.1-10 |
| tetrahydro-2-isobutyl-4-methylpyran-4-ol, mixed isomers (cis and trans) | 63500-71-0 | 1 - 3 | 1-3 | 1-3 |
| benzyl acetate | 140-11-4 | 0.25-2.5 | --- | 0.25-2.5 |
| 3,7-dimethyloctan-3-yl acetate | 20780-48-7 | 1 - 2.5 | --- | 1-2.5 |
| alfa-methyl-1,3-benzodioxole-5-propionaldehyde | 1205-17-0 | 0.25-2.5 | 1-2.5 | 0.25-2.5 |
| 2,6-di-tert-butyl-p-cresol | 128-37-0 | 0.25-2.5 | 0.25-2.5 | 0.25-2.5 |
| 3-p-cumenyl-2-methylpropionaldehyde (cyclamen aldehyde) | 103-95-7 | 0,25 - 1 | 0,25 - 1 | - |
| geranyl acetate | 105-87-3 | 0.1-0.25 | 0 - 0,25 | 0.1-2.5 |
| 6-methoxy-2,6-dimethylheptan-1-al | 62439-41-2 | 0.1 - 10 | 0,1-1 | 0.1-10 |
| 2,6-dimethyl-2-octyl acetate | 68480-08-0 | | 1-2,5 | |
| anisaldehyde | 123-11-5 | | 0-10 | |
| hexyl acetate | 142-92-7 | | 0,25-2,5 | |
| citronellyl acetate | 154-84-5 | | 0-0,25 | |
| p-menth-1-en-8-ol | 98-55-5 | | | 3-5 |
| Trimethylbenzenepropanol | 103694-68-4 | | | 0.1-0.25 |

The Invention will be described now by way of examples but is not limited to these examples.

The odor control system can comprise also other odor control mixture or systems to help to reduce the odor.

### EXAMPLES 1-3

For proving the effect on odor reduction several test samples have been prepared, which are all being exposed to real urine.

Real urine from 5 individual has been collected for the test. Odor evaluation was performed by female and male "sniffers".

The odour control mixture was applied on the acquisition layer with a syringe, more particularly various drops on 4 positions, approximatively 7.5 mg for each location to obtain a total of 30 mg x product. The drops have been applied on the central part with a centre distance between the droplets of about 20 mm. Total application 60 mm in MD.

The product with a length of 275 mm (reference or product with the active Normal size described above) is placed in an aluminum pan of dimensions 30 x 14 cm height 10 cm (or such to contain the product to be tested).

10 g of urine are added in an area of 3 cm (width) x 4 cm (length) to the centre of the product.

The pan is closed with a perforated aluminum foil.

Successively the samples are put in a stove for 7 hours at 37 degrees ± 2 degrees and then extracted from the oven and cooled.

The sniff test is performed on at least three replicates of Reference and at least three identical samples for each composition and each reference or sample is sniffed once from at least four sniffers.

The tests are carried out on products of normal production comfort pads.

The pads comprise:
- Coverstock = Air through Bonded bicomponent polyolefin NW 20 g/sqm
- Layer between topsheet and core = Air through Bonded PP 40 g/sqm
- Absorbent core = cellulose Fluff mixed with Superabsorbent polymer (total bw 613 gsm. SAP bw 160gsm)
- Backsheet : Textile breathable film 25 gsm

Data have been analysed with ANOVA. Means have been compared with t-test p< 0.05

The same test has been performed on two products containing three different odor mixtures and on a third product containing two different odor mixtures.

In table 2 the three tested compositions are reported.

**Table 2**

| Chemical Name | CAS-No. | Comp. 1 % wt. | Comp. 2 % wt. | Comp.3 % wt. |
|---|---|---|---|---|
| 2-phenylethanol | 60-12-8 | 4.26 | 4.2 | 4.3 |
| Terpineol | 8000-41-7 | 3.09 | 3.1 | |
| 3,7-dimethyloctan-3-ol | 78-69-3 | 2.78 | 3.3 | 3.5 |
| piperonal (heliotropine) | 120-57-0 | 2.47 | | 2.5 |
| tetrahydro-2-isobutyl-4-methylpyran-4-ol, mixed isomers (cis and trans) | 63500-71-0 | 1.95 | 2 | 2 |
| benzyl acetate | 140-11-4 | 1.45 | | 1.5 |
| 3,7-dimethyloctan-3-yl acetate | 20780-48-7 | 1.17 | | 1.2 |
| alfa-methyl-1,3-benzodioxole-5-propionaldehyde | 1205-17-0 | 1.03 | 1 | 1 |
| 2,6-di-tert-butyl-p-cresol | 128-37-0 | 0.71 | 0.7 | 0.7 |
| 3-p-cumenyl-2-methylpropionaldehyde (cyclamen aldehyde) | 103-95-7 | 0.63 | 0.6 | |
| geranyl acetate | 105-87-3 | 0.15 | 0.15 | 0.2 |
| 6-methoxy-2,6-dimethylheptan-1-al | 62439-41-2 | 0.15 | 0.2 | 0.2 |
| 2,6-dimethyl-2-octyl acetate | 68480-08-0 | | 1.4 | |
| anisaldehyde | 123-11-5 | | 2.5 | |
| hexyl acetate | 142-92-7 | | 1.1 | |
| citronellyl acetate | 154-84-5 | | 0.15 | |
| p-menth-1-en-8-ol | 98-55-5 | | | 3.3 |
| Trimethylbenzenepropanol | 103694-68-4 | | | 0.15 |

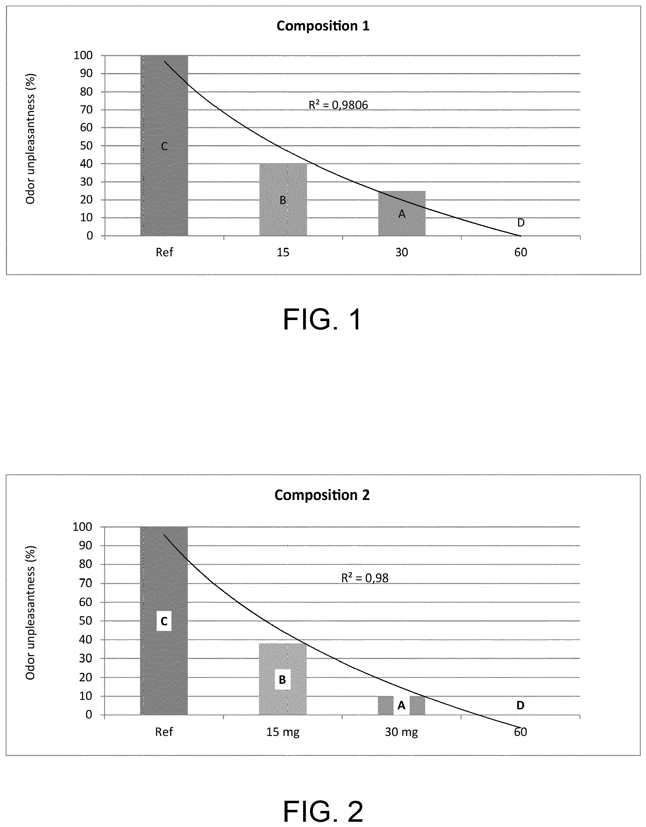

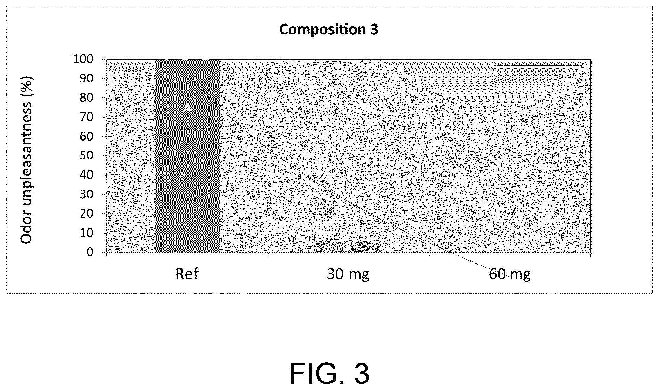

The collected data are shown in Figures 1 to 3 and show that the suppression of odor is already relevant when an odor mixture composition of only 15 mg is used and become optimal with 30 mg.

## Claims

1. An absorbent article comprising an odor control mixture comprising:
- at least one alcohol or phenol selected in the group a) consisting of 2-phenylethanol, terpineol, 3,7-dimethyloctan-3-ol, tetrahydro-2-isobutyl-4-methylpyran-4-ol, 2,6-di-tert-butyl-p-cresol, p-menth-1-en-8-ol, trimethylbenzenepropanol;
- at least one aldehyde selected in the group b) consisting of piperonal, alfa-methyl-1,3-benzodioxole-5-propionaldehyde, 3-p-cumenyl-2-methylpropionaldehyde, 6-methoxy-2,6-dimethylheptan-1-al, anisaldheyde;
- at least one acetate selected in the group c) consisting of benzyl acetate, 3,7-dimethyloctan-3-yl acetate, geranyl acetate, hexyl acetate, 2, 6-dimethyl-2-octyl acetate, citronellyl acetate.

2. The disposable absorbent article of claim 1, **characterized in that** said odor control mixture comprises:
- at least two alcohols or phenols selected in the group a),
- at least two aldehydes selected in the group b),
- at least two acetates selected in the group c).

3. The disposable absorbent article of claim 1, **characterized in that** said odor control mixture comprises:
- at least four alcohols or phenol selected in the group a),
- at least two aldehydes selected in the group b),
- at least three acetates selected in the group c).

4. The disposable absorbent article of any of the previous claims, **characterized in that** it comprises two or more alcohols or phenol selected in the group consisting of 2-phenylethanol, 3,7-dimethyloctan-3-ol, tetrahydro-2-isobutyl-4-methylpyran-4-ol mixed isomers (cis and trans), 2,6-di-tert-butyl-p-cresol, p-menth-1-en-8-ol, trimethylbenzenepropanol.

5. The disposable absorbent article of any of the previous claims, **characterized in that** it comprises the four alcohols or phenol 2-phenylethanol, 3,7-dimethyloctan-3-ol, tetrahydro-2-isobutyl-4-methylpyran-4-ol mixed isomers (cis and trans), 2,6-di-tert-butyl-p-cresol.

6. The disposable absorbent article of any of the previous claims, **characterized in that** it comprises the aldehydes alfa-methyl-1,3-benzodioxole-5-propionaldehyde, 6-methoxy-2,6-dimethylheptan-1-al.

7. The disposable absorbent article of any of the previous claims, **characterized in that** it comprises geranyl acetate.

8. The disposable absorbent article of any of the previous claims, **characterized in that** it comprises 2-phenylethanol, 3,7-dimethyloctan-3-ol, piperonal, tetrahydro-2-isobutyl-4-methylpyran-4-ol mixed isomers (cis and trans), benzyl acetate, 3,7-dimethyloctan-3-yl acetate, alfa-methyl-1,3-benzodioxole-5-propionaldehyde, 2,6-di-tert-butyl-p-cresol, geranyl acetate, 6-methoxy-2,6-dimethylheptan-1-al, p-menth-1-en-8-ol, Trimethylbenzenepropanol.

9. The disposable absorbent article of claim 1, **characterized in that** said odor control mixture comprises 2-phenylethanol, Terpineol, 3,7-dimethyloctan-3-ol, piperonal (heliotropine), tetrahydro-2-isobutyl-4-methylpyran-4-ol, benzyl acetate, 3,7-dimethyloctan-3-yl acetate, alfa-methyl-1,3-benzodioxole-5-propionaldehyde, 2,6-di-tert-butyl-p-cresol, 3-p-cumenyl-2-methylpropionaldehyde (cyclamen aldehyde), geranyl acetate, 6-methoxy-2,6-dimethylheptan-1-al.

10. The disposable absorbent article of claim 1, **characterized in that** said odor control mixture comprises: 2-phenylethanol, Terpineol, 3,7-dimethyloctan-3-ol, tetrahydro-2-isobutyl-4-methylpyran-4-ol mixed isomers (cis and trans), alfa-methyl-1,3-benzodioxole-5-propionaldehyde, 2,6-di-tert-butyl-p-cresol, 3-p-cumenyl-2-methylpropionaldehyde (cyclamen aldehyde), geranyl acetate, 6-methoxy-2,6-dimethylheptan-1-al, 2,6-dimethyl-2-octyl acetate, anisaldehyde, hexyl acetate, citronellyl acetate.

11. The disposable absorbent article of any of the preceding claims, **characterized in that** the percentage quantity in weight of the alcohols or phenols of group a) in the odor control mixture is greater than the percentage quantity of the aldehydes of group b) or the percentage quantity of the acetates of group c).

12. The disposable absorbent article of any of the preceding claims, **characterized in that** the percentage quantity in weight of the alcohols of group a) is between 8 and 24 %, the percentage quantity of the aldehydes of group b) is between 1 and 23 % and the percentage quantity of the acetates of group c) is between 1 and 8 %.

13. The disposable absorbent article of any of the preceding claims, **characterized in that** the relative percentages of groups a), b) and c) are between 40 and 90 for groups a), 3 and 45 for groups b) and 5 and 20 for groups c) .

14. The disposable absorbent article of any of the preceding claims, **characterized in that** the relative percentages of groups a), b) and c) are between 60 and 70 for groups a), 15 and 25 for groups b) and 10 and 20 for groups c).

15. The absorbent article of claim 1, **characterized in that** such absorbent article is sanitary napkins, panty liners, adult incontinence articles, diapers, paper towels, tampon, interlabial products, perspiration pads, training pants, incontinence underwear, incontinence pull-ons, bed underpad.

## Patentansprüche

1. Absorbierender Gegenstand, enthaltend eine Duftsteuermischung, enthaltend:
- zumindest einen Alkohol oder Phenol, ausgewählt aus der Gruppe a) bestehend aus 2-Phenylethanol, Terpineol, 3,7-Dimethyloctan-3-ol, Tetrahydro-2-isobutyl-4-methylpyran-4-ol, 2,6-Di-tert-butyl-p-cresol, p-Menth-1-en-8-ol, Trimethylbenzolpropanol;
- zumindest ein Aldehyd, ausgewählt aus der Gruppe b) bestehend aus Piperonal, Alfa-methyl-1,3-benzodioxol-5-propionaldehyd, 3-p-Cumenyl-2-methylpropionaldehyd, 6-Methoxy-2,6-dimethylheptan-1-al, Anisaldehyd;
- zumindest ein Acetat, ausgewählt aus der Gruppe c) bestehend aus Benzylacetat, 3,7-Dimethyloctan-3-yl-acetat, Geranylacetat, Hexylacetat, 2,6-Dimethyl-2-octylacetat, Citronellylacetat.

2. Wegwerfbarer absorbierender Gegenstand gemäß Anspruch 1, **dadurch gekennzeichnet, dass** die Duftsteuermischung enthält:
- zumindest zwei Alkohole oder Phenole, ausgewählt aus der Gruppe a),
- zumindest zwei Aldehyde, ausgewählt aus der Gruppe b),
- zumindest zwei Acetate, ausgewählt aus der Gruppe c).

3. Wegwerfbarer absorbierender Gegenstand gemäß Anspruch 1, **dadurch gekennzeichnet, dass** die Duftsteuermischung enthält:
- zumindest vier Alkohole oder Phenol, ausgewählt aus der Gruppe a),
- zumindest zwei Aldehyde, ausgewählt aus der Gruppe b),
- zumindest drei Acetate, ausgewählt aus der Gruppe c).

4. Wegwerfbarer absorbierender Gegenstand gemäß einem der vorhergehenden Ansprüche, **dadurch gekennzeichnet, dass** er zwei oder mehrere Alkohole oder Phenol enthält, ausgewählt aus der Gruppe bestehend aus 2-Phenylethanol, 3,7-Dimethyloctan-3-ol, Tetrahydro-2-isobutyl-4-methylpyran-4-ol-gemischte Isomere (cis und trans), 2,6-Di-tert-butyl-p-cresol, p-Menth-1-en-8-ol, Trimethylbenzolpropanol.

5. Wegwerfbarer absorbierender Gegenstand gemäß einem der vorhergehenden Ansprüche, **dadurch gekennzeichnet, dass** er die vier Alkohole oder Phenol 2-Phenylethanol, 3,7-Dimethyloctan-3-ol, Tetrahydro-2-isobutyl-4-methylpyran-4-ol-gemischte Isomere (cis und trans), 2,6-Di-tert-butyl-p-cresol enthält.

6. Wegwerfbarer absorbierender Gegenstand gemäß einem der vorhergehenden Ansprüche, **dadurch gekennzeichnet, dass** er die Aldehyde Alfa-methyl-1,3-benzodioxol-5-propionaldehyd, 6-Methoxy-2,6-dimethylheptan-1-al enthält.

7. Wegwerfbarer absorbierender Gegenstand gemäß einem der vorhergehenden Ansprüche, **dadurch gekennzeichnet, dass** er Geranylacetat enthält.

8. Wegwerfbarer absorbierender Gegenstand gemäß einem der vorhergehenden Ansprüche, **dadurch gekennzeichnet, dass** er 2-Phenylethanol, 3,7-Dimethyloctan-3-ol, Piperonal, Tetrahydro-2-isobutyl-4-methylpyran-4-ol-gemischte Isomere (cis und trans), Benzylacetat, 3,7-Dimethyloctan-3-yl-acetat, Alfa-methyl-1,3-benzodioxol-5-propionaldehyd, 2,6-Di-tert-butyl-p-cresol, Geranylacetat, 6-Methoxy-2,6-dimethylheptan-1-al, p-Menth-1-en-8-ol, Trimethylbenzolpropanol enthält.

9. Wegwerfbarer absorbierender Gegenstand gemäß Anspruch 1, **dadurch gekennzeichnet, dass** die Duftsteuermischung 2-Phenylethanol, Terpineol, 3,7-Dimethoyloctan-3-ol, Piperonal (Heliotropin), Tetrahydro-2-isobutyl-4-methylpyran-4-ol, Benzylacetat, 3,7-Dimethyloctan-3-yl-acetat, Alfa-methyl-1,3-benzodioxol-5-propionaldehyd, 2,6-Di-tert-butyl-p-cresol, 3-p-Cumenyl-2-methylpropionaldehyd (Cyclamenaldehyd), Geranylacetat, 6-Methoxy-2,6-dimethylheptan-1-al enthält.

10. Wegwerfbarer absorbierender Gegenstand gemäß Anspruch 1, **dadurch gekennzeichnet, dass** die Duftsteuermischung enthält: 2-Phenylethanol, Terpineol, 3,7-Dimethoyloctan-3-ol, Tetrahydro-2-isobutyl-4-methylpyran-4-ol-gemischte Isomere (cis und trans), Alfa-methyl-1,3-benzodioxol-5-propionaldehyd, 2,6-Di-tert-butyl-p-cresol, 3-p-Cumenyl-2-methylpropionaldehyd (Cyclamenaldehyd), Geranylacetat, 6-Methoxy-2,6-dimethylheptan-1-al, 2,6-Dimethyl-2-octylacetat, Anisaldehyd, Hexylacetat, Citronellylacetat.

11. Wegwerfbarer absorbierender Gegenstand gemäß einem der vorhergehenden Ansprüche, **dadurch gekennzeichnet, dass** der Prozentsatz als Geweicht der Alkohole oder Phenole der Gruppe a) in der Duftsteuermischung größer ist als der Prozentsatz der Menge der Aldehyde der Gruppe b) oder der Prozentsatz der Menge der Acetate der Gruppe c) .

12. Wegwerfbarer absorbierender Gegenstand gemäß einem der vorhergehenden Ansprüche, **dadurch gekennzeichnet, dass** der Prozentsatz der Menge des Geweichtes der Alkohole der Gruppe a) zwischen 8 und 24 %, der Prozentsatz der Menge der Aldehyde der Gruppe b) zwischen 1 und 23 % und der Prozentsatz der Menge der Acetate der Gruppe c) zwischen 1 und 8 % ist.

13. Wegwerfbarer absorbierender Gegenstand gemäß einem der vorhergehenden Ansprüche, **dadurch gekennzeichnet, dass** die relativen Prozentsätze der Gruppen a), b) und c) zwischen 40 und 90 für die Gruppen a), 3 und 45 für die Gruppen b) und 5 bis 20 für die Gruppen c) sind.

14. Wegwerfbarer absorbierender Gegenstand gemäß einem der vorhergehenden Ansprüche, **dadurch gekennzeichnet, dass** die relativen Prozentsätze der Gruppen a), b) und c) zwischen 60 und 70 für die Gruppen a), 15 und 25 für die Gruppen b) und 10 und 20 für die Gruppen c) sind.

15. Wegwerfbarer absorbierender Gegenstand gemäß Anspruch 1, **dadurch gekennzeichnet, dass** ein solcher absorbierender Gegenstand eine Damenbinde, Slip-Einlage, Inkontinenzgegenstand für Erwachsene, Windeln, Papiertücher, Tampons, interlabiale Produkte, Schweißpads, Trainingshosen, Inkontinenz-Unterwäsche, Inkontinenz-Schlupfhosen, Bettunterlagen ist.

## Revendications

1. Article absorbant comprenant un mélange de contrôle des odeurs comprenant :
- au moins un alcool ou phénol choisi dans le groupe a) constitué des 2-phényléthanol, terpinéol, 3,7-diméthyloctan-3-ol, tétrahydro-2-isobutyl-4-méthylpyran-4-ol, 2,6-di-tert-butyl-p-crésol, p-menth-1-én-8-ol, triméthylbenzènepropanol ;
- au moins un aldéhyde choisi dans le groupe b) constitué des pipéronal, alpha-méthyl-1,3-benzodioxole-5-propionaldéhyde, 3-p-cuményl-2-méthylpropionaldéhyde, 6-méthoxy-2,6-diméthylheptan-1-al, anisaldéhyde ;
- au moins un acétate choisi dans le groupe c) constitué des acétate de benzyle, acétate de 3,7-diméthyloctan-3-yle, acétate de géranyle, acétate d'hexyle, acétate de 2,6-diméthyl-2-octyle, acétate de citronellyle.

2. Article absorbant jetable selon la revendication 1, **caractérisé en ce que** ledit mélange de contrôle des odeurs comprend :
- au moins deux alcools ou phénols choisis dans le groupe a),
- au moins deux aldéhydes choisis dans le groupe b),
- au moins deux acétates choisis dans le groupe c).

3. Article absorbant jetable selon la revendication 1, **caractérisé en ce que** ledit mélange de contrôle des odeurs comprend :
- au moins quatre alcools ou phénols choisis dans le groupe a),
- au moins deux aldéhydes choisis dans le groupe b),
- au moins trois acétates choisis dans le groupe c).

4. Article absorbant jetable selon l'une quelconque des revendications précédentes, **caractérisé en ce qu'**il comprend deux ou plus de deux alcools ou phénols choisis dans le groupe constitué des 2-phényléthanol, 3,7-diméthyloctan-3-ol, mélange d'isomères (cis et trans) de tétrahydro-2-isobutyl-4-méthylpyran-4-ol, 2,6-di-tert-butyl-p-crésol, p-menth-1-én-8-ol, triméthylbenzènepropanol.

5. Article absorbant jetable selon l'une quelconque des revendications précédentes, **caractérisé en ce qu'**il comprend les quatre alcools ou phénols 2-phényléthanol, 3,7-diméthyloctan-3-ol, mélange d'isomères (cis et trans) de tétrahydro-2-isobutyl-4-méthylpyran-4-ol, 2,6-di-tert-butyl-p-crésol.

6. Article absorbant jetable selon l'une quelconque des revendications précédentes, **caractérisé en ce qu'**il comprend les aldéhydes alpha-méthyl-1,3-benzodioxole-5-propionaldéhyde, 6-méthoxy-2,6-diméthylheptan-1-al.

7. Article absorbant jetable selon l'une quelconque des revendications précédentes, **caractérisé en ce qu'**il comprend de l'acétate de géranyle.

8. Article absorbant jetable selon l'une quelconque des revendications précédentes, **caractérisé en ce qu'**il comprend du 2-phényléthanol, 3,7-diméthyloctan-3-ol, pipéronal, mélange d'isomères (cis et trans) de tétrahydro-2-isobutyl-4-méthylpyran-4-ol, acétate de benzyle, acétate de 3,7-diméthyloctan-3-yle, alpha-méthyl-1,3-benzodioxole-5-propionaldéhyde, 2,6-di-tert-butyl-p-crésol, acétate de géranyle, 6-méthoxy-2,6-diméthylheptan-1-al, p-menth-1-én-8-ol, triméthylbenzènepropanol.

9. Article absorbant jetable selon la revendication 1, **caractérisé en ce que** ledit mélange de contrôle des odeurs comprend du 2-phényléthanol, terpinéol, 3,7-diméthyloctan-3-ol, pipéronal (héliotropine), tétrahydro-2-isobutyl-4-méthylpyran-4-ol, acétate de benzyle, acétate de 3,7-diméthyloctan-3-yle, alpha-méthyl-1,3-benzodioxole-5-propionaldéhyde, 2,6-di-tert-butyl-p-crésol, 3-p-cuményl-2-méthylpropionaldéhyde (cyclaménaldéhyde), acétate de géranyle, 6-méthoxy-2,6-diméthylheptan-1-al.

10. Article absorbant jetable selon la revendication 1, **caractérisé en ce que** ledit mélange de contrôle des odeurs comprend les : 2-phényléthanol, terpinéol, 3,7-diméthyloctan-3-ol, mélange d'isomères (cis et trans) de tétrahydro-2-isobutyl-4-méthylpyran-4-ol, alpha-méthyl-1,3-benzodioxole-5-propionaldéhyde, 2,6-di-tert-butyl-p-crésol, 3-p-cuményl-2-méthylpropionaldéhyde (cyclaménaldéhyde), acétate de géranyle, 6-méthoxy-2,6-diméthylheptan-1-al, acétate de 2,6-diméthyl-2-octyle, anisaldéhyde, acétate d'hexyle, acétate de citronellyle.

11. Article absorbant jetable selon l'une quelconque des revendications précédentes, **caractérisé en ce que** la quantité en pourcentage en poids des alcools ou phénols du groupe a) dans le mélange de contrôle des odeurs est supérieure à la quantité en pourcentage des aldéhydes du groupe b) ou la quantité en pourcentage des acétates du groupe c).

12. Article absorbant jetable selon l'une quelconque des revendications précédentes, **caractérisé en ce que** la quantité en pourcentage en poids des alcools de groupe a) est comprise entre 8 et 24 %, la quantité en pourcentage des aldéhydes du groupe b) est comprise entre 1 et 23 % et la quantité en pourcentage des acétates du groupe c) est comprise entre 1 et 8 %.

13. Article absorbant jetable selon l'une quelconque des revendications précédentes, **caractérisé en ce que** les pourcentages relatifs des groupes a), b) et c) sont compris entre 40 et 90 pour les groupes a), 3 et 45 pour les groupes b) et 5 et 20 pour les groupes c).

14. Article absorbant jetable selon l'une quelconque des revendications précédentes, **caractérisé en ce que** les pourcentages relatifs des groupes a), b) et c) sont compris entre 60 et 70 pour les groupes a), 15 et 25 pour les groupes b) et 10 et 20 pour les groupes c).

15. Article absorbant selon la revendication 1, **caractérisé en ce qu'**un tel article absorbant est : des serviettes hygiéniques, des protège-slips, des articles pour incontinence pour adultes, des couches, des serviettes en papier, des tampons, des produits interlabiaux, des tampons antitranspiration, des sous-vêtements d'entraînement, des sous-vêtements pour incontinence, des culottes pour incontinence, une alaise.
